# EUROPÄISCHE PATENTSCHRIFT

(11) **EP 3 041 917 B1**
(45) Veröffentlichungstag und Bekanntmachung des Hinweises auf die Patenterteilung: **25.10.2017**
(21) Anmeldenummer: 14761336.8
(22) Anmeldetag: 04.09.2014
(51) Int. Cl.: C10G 45/58, C10G 65/04, C10G 69/06, C10G 9/36, C10G 70/04, C07C 5/13, C07C 11/08, C07C 5/27

(54) **VERFAHREN ZUR ERZEUGUNG VON KOHLENWASSERSTOFFPRODUKTEN**
METHOD FOR PRODUCING HYDROCARBON PRODUCTS
PROCÉDÉ DE PRODUCTION D'HYDROCARBURES

(30) Priorität: 05.09.2013 DE 102013014867; 05.09.2013 DE 102013014802; 25.09.2013 EP 13004660; 25.09.2013 EP 13004661
(43) Veröffentlichungstag der Anmeldung: 13.07.2016
(73) Patentinhaber: Linde Aktiengesellschaft, 80331 München (DE)
(72) Erfinder: SCHMIDT, Gunther, 82041 Deisenhofen (DE); DR. WALTER, Stefanie, 82418 Seehausen (DE); FRITZ, Helmut, 81375 München (DE)
(86) Internationale Anmeldenummer: PCT/EP2014/068842
(87) Internationale Veröffentlichungsnummer: WO 2015/032860

(56) Entgegenhaltungen:
- EP-A1- 2 062 865
- DE-A1- 2 805 179
- US-A- 3 922 216
- US-A- 4 091 046
- US-A- 5 523 502
- US-A1- 2011 112 345
- US-B2- 6 743 958

## Beschreibung

Die Erfindung betrifft ein Verfahren zur Erzeugung von Kohlenwasserstoffprodukten.

### Stand der Technik

Verfahren und Vorrichtungen zum Dampfspalten (engl. Steam Cracking) von Kohlenwasserstoffen sind bekannt und beispielsweise im Artikel "Ethylene" in Ullmann's Encyclopedia of Industrial Chemistry, online seit 15. April 2007, DOI 10.1002/14356007.a10_045.pub2, beschrieben. Die US 2011/112345 A1 offenbart ein Verfahren zur Erzeugung von Olefinen enthaltend Ethylen und Propylen. Die US 3,922,216 A offenbart ein Verfahren, bei dem ein Kohlenwasserstoffstrom, der überwiegend Kohlenwasserstoffe mit zwei bis fünf Kohlenstoffatomen enthält, nach der Entfernung von Isobutan mit einem Kohlenwasserstoffstrom vereinigt wird, der Kohlenwasserstoffe mit neun und mehr Kohlenstoffatomen enthält. Der dabei gebildete Strom wird einem Dampfspaltverfahren unterworfen. Die EP 2 062 865 A1 offenbart ein Verfahren zur Herstellung von Ethylen, Propylen und Isopren aus leichten Kohlenwasserstoffen, bei dem aus einer Butanfraktion, die optional Ethan enthalten kann, Isobutan abgetrennt wird. Der Rest kann in einer oder mehreren Spaltzonen in einem Dampfspaltverfahren umgesetzt werden. Gattungsgleiche Verfahren sind in der DE 28 05 179 A1, der US 6,743,958 B2, der US 5,523,502 A, der US 2011/112345 A1 und der US 4,091,046 A angegeben.

In neueren Verfahren und Vorrichtungen zum Dampfspalten kommen zunehmend milde Spaltbedingungen zum Einsatz (siehe unten), weil sich bei diesen insbesondere sogenannte Wertprodukte, beispielsweise Propylen und Butadien, mit verbesserter Ausbeute gewinnen lassen, wie unten erläutert. Gleichzeitig verringert sich bei milden Spaltbedingungen jedoch auch die Umsetzung des Ofeneinsatzes, so dass sich in diesem enthaltene Verbindungen in vergleichsweise großer Menge im Spaltgas wiederfinden und zu einer "Verdünnung" der Wertprodukte führen.

Aufgabe vorliegender Erfindung ist es, hier Abhilfe zu schaffen und unter Beibehaltung der Vorteile der milden Spaltbedingungen deren Nachteile zu vermeiden. Insbesondere soll durch eine Reduzierung des erwähnten Verdünnungseffekts die Konzentration und Menge der Wertprodukte, insbesondere von 1,3-Butadien, erhöht werden.

### Offenbarung der Erfindung

Diese Aufgabe wird durch ein Verfahren zur Erzeugung von Kohlenwasserstoffprodukten mit den Merkmalen des Anspruchs 1 gelöst. Bevorzugte Ausgestaltungen sind Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Dampfspaltverfahren werden im kommerziellen Maßstab nahezu ausschließlich in Rohrreaktoren durchgeführt, in denen einzelne Reaktionsrohre (in Form von Rohrschlangen, sogenannten Coils) oder Gruppen von entsprechenden Reaktionsrohren auch bei unterschiedlichen Spaltbedingungen betrieben werden können. Unter gleichen oder vergleichbaren Spaltbedingungen betriebene Reaktionsrohre oder Gruppen von Reaktionsrohren, gegebenenfalls aber auch unter einheitlichen Spaltbedingungen betriebene Rohrreaktoren insgesamt, werden nachfolgend jeweils als "Spaltöfen" bezeichnet. Ein Spaltofen ist im hier verwendeten Sprachgebrauch also eine zum Dampfspalten verwendete bauliche Einheit, die einen Ofeneinsatz gleichen oder vergleichbaren Spaltbedingungen aussetzt. Eine Anlage zum Dampfspalten kann einen oder mehrere derartiger Spaltöfen aufweisen.

Mit dem Begriff "Ofeneinsatz" werden hier ein oder mehrere flüssige und/oder gasförmige Ströme bezeichnet, die einem oder mehreren Spaltöfen zugeführt werden. Auch durch ein entsprechendes Dampfspaltverfahren erhaltene Ströme, wie unten erläutert, können in einen oder mehrere Spaltöfen zurückgeführt und erneut als Ofeneinsatz verwendet werden. Als Ofeneinsatz eignet sich eine Vielzahl von Kohlenwasserstoffen und Kohlenwasserstoffgemischen von Ethan bis Gasöl bis zu einem Siedepunkt von typischerweise 600 °C.

Ein Ofeneinsatz kann aus einem sogenannten "Frischeinsatz" bestehen, also aus einem Einsatz, der anlagenextern bereitgestellt und beispielsweise aus einer oder mehreren Erdölfraktionen, Erdgaskomponenten mit zwei bis vier Kohlenstoffatomen und/oder Erdgaskondensaten gewonnen wird. Ein Ofeneinsatz kann auch aus einem oder mehreren sogenannten "Recycleströmen" bestehen, also Strömen, die in der Anlage selbst erzeugt und in einen entsprechenden Spaltofen zurückgeführt werden. Ein Ofeneinsatz kann auch aus einem Gemisch eines oder mehrerer Frischeinsätze mit einem oder mehreren Recycleströmen bestehen.

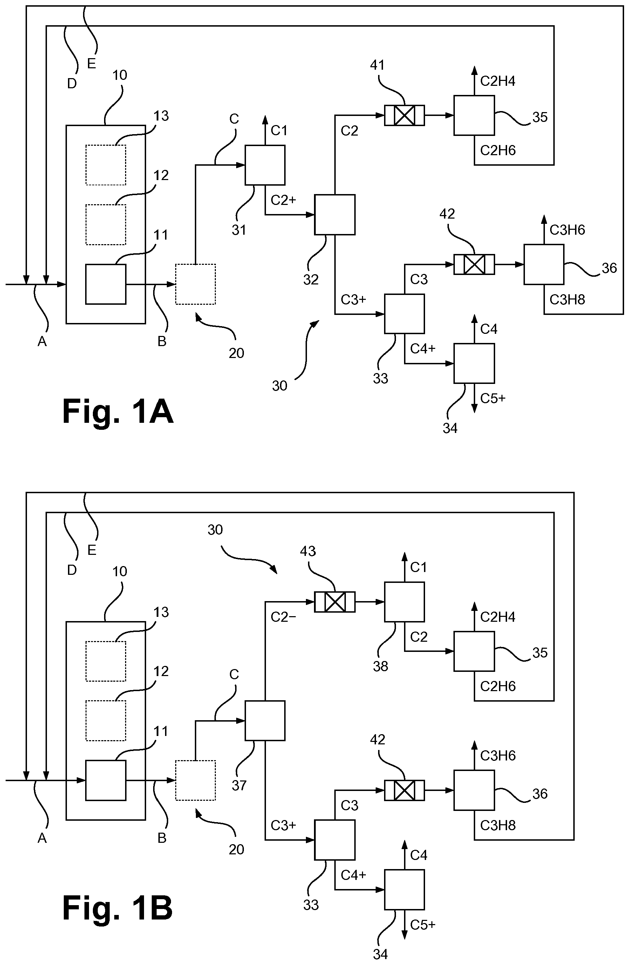

Der Ofeneinsatz wird im jeweiligen Spaltofen zumindest teilweise umgesetzt und verlässt den Spaltofen als sogenanntes "Rohgas", das, wie unten unter Bezugnahme auf die Figuren 1A und 1B erläutert, einer Reihe von Nachbehandlungsschritten unterworfen werden kann. Derartige Nachbehandlungsschritte umfassen zunächst eine Aufbereitung des Rohgases, beispielsweise durch Quenchen, Kühlen und Trocknen, wodurch ein sogenanntes "Spaltgas" erhalten wird. Bisweilen wird auch bereits das Rohgas als Spaltgas bezeichnet.

Gängige Verfahren umfassen insbesondere die Trennung des Spaltgases in eine Reihe von Fraktionen auf Grundlage der unterschiedlichen Siedepunkte der enthaltenen Komponenten. In der Fachwelt werden hierfür Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (jedoch konventionsgemäß unter Umständen auch Wasserstoff, dann auch "C1 minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend Propan, Propylen, Methylacetylen und/oder Propadien. Eine "C4-Fraktion" enthält überwiegend oder ausschließlich Butan, Buten, Butadien und/oder Butin, wobei die jeweiligen Isomere je nach Quelle der C4-Fraktion in unterschiedlichen Anteilen enthalten sein können. Entsprechendes gilt auch für eine "C5-Fraktion" und die höheren Fraktionen. Mehrere solcher Fraktionen können auch verfahrens- und/oder bezeichnungsmäßig zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem oder zwei Kohlenstoffatomen.

Flüssige und gasförmige Ströme können im hier verwendeten Sprachgebrauch reich oder arm an einer oder mehreren Komponenten sein, wobei "reich" für einen Gehalt von wenigstens 90%, 95%, 99%, 99,5%, 99,9%, 99,99% oder 99,999% und "arm" für einen Gehalt von höchstens 10%, 5%, 1%, 0,1%, 0,01% oder 0,001% auf molarer, Gewichts- oder Volumenbasis stehen kann. Der Begriff "überwiegend" bezeichnet einen Gehalt von wenigstens 50%, 60%, 70%, 80% oder 90% oder entspricht dem Begriff "reich". Flüssige und gasförmige Ströme können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem der flüssige oder gasförmige Strom erhalten wurde. Der flüssige oder gasförmige Strom ist "angereichert", wenn dieser zumindest den 1,1-fachen, 1,5-fachen, 2-fachen, 5-fachen, 10-fachen, 100-fachen oder 1.000-fachen Gehalt, "abgereichert", wenn er höchstens den 0,9-fachen, 0,5-fachen, 0,1-fachen, 0,01-fachen oder 0,001-fachen Gehalt einer entsprechenden Komponente, bezogen auf das Ausgangsgemisch, enthält.

Ein Strom kann von einem anderen Strom "abgeleitet" sein, beispielsweise durch Verdünnung, Aufkonzentrierung, Anreicherung, Abreicherung, Abtrennung oder Umsetzung beliebiger Komponenten, durch Trennschritte oder auch durch eine Vereinigung mit wenigstens einem weiteren Strom. Ein abgeleiteter Strom kann auch durch Aufteilen eines Ausgangsstroms in wenigstens zwei Teilströme gebildet werden, wobei dann jeder Teilstrom oder auch ein Reststrom nach Abtrennung eines anderen Stroms ein solcher abgeleiteter Strom ist.

Die erwähnten "Spaltbedingungen" in einem Spaltofen umfassen unter anderem den Partialdruck des Ofeneinsatzes, der durch die Zugabe unterschiedlicher Dampfmengen und den im Spaltofen eingestellten Druck beeinflusst werden kann, die Verweildauer im Spaltofen sowie die in diesem verwendeten Temperaturen und Temperaturprofile. Auch die Ofengeometrie und Ofengestaltung spielt eine Rolle. Zur Herstellung von Ethylen wird ein Spaltofen beispielsweise bei einer Ofeneintrittstemperatur von 500 bis 680°C und bei einer Ofenaustrittstemperatur von 775 bis 875°C betrieben. Hierbei ist die "Ofeneintrittstemperatur" die Temperatur eines Gasstroms am Beginn eines Reaktionsrohrs und die "Ofenaustrittstemperatur" die Temperatur eines Gasstroms am Ende eines Reaktionsrohrs. Typischerweise handelt es sich bei letzterer um die maximale Temperatur, auf die der entsprechende Gasstrom aufgeheizt wird. Dem Ofeneinsatz wird dabei bei einem, ebenfalls am Ofenausgang gemessenen, Druck von 165 bis 225 kPa Dampf in einem Verhältnis von typischerweise 0,25 bis 0,85 kg/kg beigemischt. Die spezifisch verwendeten Werte sind vom jeweils verwendeten Ofeneinsatz und den erwünschten Spaltprodukten abhängig.

Da die genannten Werte einander zumindest teilweise wechselseitig beeinflussen, hat sich zur Charakterisierung der Spaltbedingungen der Begriff der "Spaltschärfe" (engl. Cracking Severity) durchgesetzt. Für flüssige Ofeneinsätze kann die Spaltschärfe über das Verhältnis von Propylen zu Ethylen (P/E) oder als das Verhältnis von Methan zu Propylen (M/P) im Spaltgas auf Gewichtsbasis (kg/kg) beschrieben werden. Das P/E- und das M/P-Verhältnis sind direkt von der Temperatur abhängig, können aber im Gegensatz zur realen Temperatur im oder am Ausgang eines Spaltofens sehr viel genauer gemessen und beispielsweise als Regelgröße in einem entsprechenden Regelungsverfahren verwendet werden. Das P/E-Verhältnis eignet sich aber nur bedingt zur Charakterisierung der Spaltschärfe bei gasförmigen Ofeneinsätzen bzw. Verbindungen mit zwei bis vier Kohlenstoffatomen.

Für gasförmige Ofeneinsätze kann die Umsetzung bzw. Konversion einer jeweils betrachteten Komponente des Ofeneinsatzes als Maß der Spaltschärfe angegeben werden. Der Begriff Umsetzung bzw. Konversion (engl. Conversion) wird hier wie in der Fachwelt üblich verwenden (siehe beispielsweise den erwähnten Artikel "Ethylene" in Ullmann's Encyclopedia of Industrial Chemistry). Insbesondere für die im vorliegenden Fall eingesetzten C4-Fraktionen bzw. C4-Teilströme ist eine Beschreibung der Spaltschärfe über die Umsetzung von Schlüsselkomponenten wie n-Butan und iso-Butan gut geeignet.

Die Spaltschärfen bzw. Spaltbedingungen sind "scharf", wenn n-Butan in einer entsprechenden Fraktion zu mehr als 92% umgesetzt wird. Bei noch schärferen Spaltbedingungen wird n-Butan ggf. auch zu mehr als 93%, 94% oder 95% umgesetzt. Eine Umsetzung von n-Butan zu 100% erfolgt typischerweise nicht. Die Obergrenze der "scharfen" Spaltschärfen bzw. Spaltbedingungen liegt daher beispielsweise bei 99%, 98%, 97% oder 96% Umsetzung von n-Butan. Die Spaltschärfen bzw. Spaltbedingungen sind hingegen "mild", wenn n-Butan zu weniger als 92% umgesetzt wird. Bei weniger als 91%, weniger als 90%, weniger als 89%, weniger als 88% oder weniger als 87% Umsetzung von n-Butan liegen zunehmend mildere Spaltschärfen bzw. Spaltbedingungen vor. Bei weniger als 86% Umsetzung von n-Butan werden die Spaltschärfen bzw. Spaltbedingungen hier als "sehr mild" bezeichnet. Sehr milde Spaltschärfen bzw. Spaltbedingungen umfassen auch beispielsweise eine Umsetzung von n-Butan zu weniger als 85%, 84%, 83%, 82%, 81%, 80%, 79%, 78%, 77%, 76%, 75%, 70% oder 65% und zu mehr als 50% oder 60%.

Die Spaltschärfen bzw. die Spaltbedingungen sind auch dann "scharf", wenn iso-Butan in einer entsprechenden Fraktion zu mehr als 91% umgesetzt wird. Bei noch schärferen Spaltbedingungen wird iso-Butan ggf. auch zu mehr als 92%, 93% oder 94% umgesetzt. Auch eine Umsetzung von iso-Butan zu 100% erfolgt typischerweise nicht. Die Obergrenze der "scharfen" Spaltschärfen bzw. Spaltbedingungen liegt daher beispielsweise bei 99%, 98%, 97% oder 96% Umsetzung von iso-Butan. Die Spaltschärfen bzw. die Spaltbedingungen sind hingegen "mild", wenn iso-Butan zu weniger als 91% umgesetzt wird. Bei weniger als 90%, weniger als 89%, weniger als 88%, weniger als 87% oder weniger als 86% Umsetzung von iso-Butan liegen zunehmend mildere Spaltschärfen bzw. Spaltbedingungen vor. Bei weniger als 83% Umsetzung von iso-Butan werden die Spaltschärfen bzw. Spaltbedingungen hier als "sehr mild" bezeichnet. Sehr milde Spaltschärfen bzw. Spaltbedingungen umfassen auch beispielsweise eine Umsetzung von iso-Butan zu weniger als 82%, 81%, 80%, 79%, 78%, 77%, 76%, 75% oder 70% und zu mehr als 45% oder 50%.

Die genannten Spaltschärfen bzw. Spaltbedingungen hängen insbesondere mit der oben erläuterten Ofenaustrittstemperatur am Ende des oder der jeweils verwendeten Reaktionsrohre bzw. Spaltöfen zusammen. Je höher diese ist, desto "schärfer", je niedriger, desto "milder" sind die Spaltschärfen bzw. Spaltbedingungen.

Ferner versteht sich, dass die Umsetzung anderer Komponenten nicht identisch mit jener des n- und des iso-Butans sein muss. Werden beispielsweise 1- und 2-Buten zusammen mit n-Butan gespalten, so werden diese typischerweise in größerem Umfang umgesetzt als das n-Butan. Umgekehrt wird iso-Buten in geringerem Umfang als iso-Butan umgesetzt, wenn es zusammen mit diesem gespalten wird. Mit einer prozentualen Umsetzung einer Schlüsselkomponente, hier n-Butan bzw. iso-Butan, sind also jeweils eine Ofenaustrittstemperatur und die jeweiligen prozentualen Umsetzungen der anderen Komponenten im Einsatz verknüpft. Diese Ofenaustrittstemperatur ist wiederum unter anderem vom Spaltofen abhängig. Der Versatz zwischen den jeweiligen prozentualen Umsetzungen ist von einer Reihe von weiteren Faktoren abhängig.

### Vorteile der Erfindung

Die vorliegende Erfindung geht von einem Verfahren zur Erzeugung von Kohlenwasserstoffprodukten aus, bei dem ein Kohlenwasserstoffstrom bereitgestellt wird, der überwiegend, d.h. mindestens 80%, verzweigte und unverzweigte Kohlenwasserstoffe mit vier Kohlenstoffatomen aufweist (nachfolgend und entsprechend dem üblichen Sprachgebrauch als C4-Fraktion oder C4-Kohlenwasserstoffstrom bezeichnet; Kurzbezeichnung und Bezugszeichen in den Figuren: C4). Insoweit entspricht das erfindungsgemäße Verfahren beispielsweise bekannten Verfahren zur Erzeugung von Kohlenwasserstoffprodukten durch Dampfspalten, bei denen aus einem Spaltgas, das gegebenenfalls aufbereitet wurde, ein solcher C4-Kohlenwasserstoffstrom abgetrennt wird. Dies kann in bekannten Anlagen in einem sogenannten Debutanizer erfolgen (der jedoch auch alle anderen Kohlenwasserstoffe mit vier Kohlenstoffatomen aus einem entsprechenden Kohlenwasserstoffstrom abtrennt). Details hierzu sind in dem erwähnten Artikel "Ethylene" in Ullmann's Encyclopedia of Industrial Chemistry gezeigt und unter Bezugnahme auf die Figuren 1A und 1B veranschaulicht.

Die Erfindung ist jedoch nicht auf die Verwendung von C4-Kohlenwasserstoffströmen beschränkt, die durch Dampfspalten und nachgeordnete Verfahrensschritte bereitgestellt werden, sondern eignet sich in gleicher Weise auch für C4-Kohlenwasserstoffströme, die zumindest teilweise mittels anderer Verfahren, beispielsweise mittels Raffinerieprozessen, erzeugt werden. Beispielsweise kann die Erfindung mit C4-Strömen verwendet werden, die zuvor nicht dampfgespalten wurden und erst anschließend einem entsprechenden Dampfspaltprozess zugeführt werden. Hierbei kann es sich beispielsweise um Erdölfraktionen, Erdgaskomponenten mit zwei bis vier Kohlenstoffatomen, Erdgaskondensate usw. oder Produkte aus Raffinerieumwandlungsprozessen handeln. Insbesondere kann ein solcher C4-Kohlenwasserstoffstrom einem oder mehreren Prozessen unterworfen werden, mittels derer in dem C4-Kohlenwasserstoffstrom enthaltene Verbindungen umgesetzt und/oder abgetrennt werden.

Die Erfindung sieht nun vor, aus diesem C4-Kohlenwasserstoffstrom oder einem hiervon abgeleiteten Strom einen Teilstrom mit überwiegend, d.h. mindestens 80 %, unverzweigten Kohlenwasserstoffen mit vier Kohlenstoffatomen (also n-C4-Verbindungen, daher als n-C4-Fraktion oder n-C4-Teilstrom bezeichnet; Kurzbezeichnung und Bezugszeichen in den Figuren: n-C4) und einen Teilstrom mit überwiegend verzweigten Kohlenwasserstoffen mit vier Kohlenstoffatomen (also iso-C4-Verbindungen, daher als iso-C4-Fraktion oder iso-C4-Teilstrom bezeichnet; Kurzbezeichnung und Bezugszeichen in den Figuren: i-C4) zu gewinnen.

Die Erfindung sieht vor, zumindest einen Teil des n-C4-Teilstroms oder eines hiervon abgeleiteten Stroms bei einer Spaltschärfe zu spalten, bei der in dem n-C4-Teilstrom enthaltenes n-Butan zu mindestens 50% und weniger als 92% umgesetzt wird. Hierbei werden also milde oder sehr milde Spaltschärfen bzw. Spaltbedingungen eingesetzt. Diese können beispielsweise auch weniger als 90%, 88%, 86%, 84%, 82%, 80%, 78%, 76%, 74%, 72%, 70% oder 65%, jedoch beispielsweise mehr als 50% oder 60% Umsetzung von n-Butan entsprechen. Bei zunehmend milden Spaltbedingungen entstehen im Verhältnis zum Frischeinsatz mehr erwünschte Produkte wie Butadien und Propylen, so dass sich die Ausbeute erhöht und das Produktspektrum verbessert.

Das "Spalten" umfasst dabei, den n-C4-Teilstrom oder einen hiervon abgeleiteten Strom (oder einen entsprechenden Anteil) alleine oder zusammen mit weiteren Strömen, gegebenenfalls auch nach voriger Vereinigung zu einem Sammelstrom, in einen Spaltofen gemäß der eingangs getroffenen Definition einzuspeisen und dem Spaltofen ein Spaltgas zu entnehmen.

In dem iso-C4-Teilstrom enthaltene iso-C4-Verbindungen werden, beispielsweise vor einer Einspeisung eines entsprechenden Teilstroms in einen Spaltofen (siehe unten) und/oder vor einer Entnahme als Produkt und/oder vor einer weiteren Auftrennung des iso-C4-Teilstroms vorteilhafterweise ferner zumindest teilweise mittels eines Skelettisomerisierungsvefahrens zu n-C4-Verbindungen umgesetzt. Ein entsprechendes Skelettisomerisierungsverfahren umfasst die Verwendung geeigneter Katalysatoren und Reaktionsbedingungen, beispielsweise wie in der US 6 743 958 B2 oder der US 6 916 448 B2 offenbart:
Eine Skelettisomerisierung kann unter Verwendung von Aluminiumoxidkatalysatoren (in denen γ-Aluminiumoxid als Adsorbens, als Katalysatorträger und/oder als Katalysator selbst Verwendung finden kann) vorgenommen werden. Hierbei können auch beispielsweise aktiviertes und/oder dampfbehandeltes Aluminiumoxid zum Einsatz kommen, wie in der US 3 558 733 A angegeben. Ferner können Titan- oder borhaltige Verbindungen verwendet werden, insbesondere in Verbindung mit η- oder γ-Aluminiumoxid, wie in der US 5 321 195 A und der US 5 659 104 A beschrieben. Weitere einsetzbare Verbindungen sind halogenierte Aluminiumoxide, wie beispielsweise in der US 2 417 647 A offenbart, Bauxite oder Zeolithe. Auch die Verwendung von mikroporös strukturierten Molekularsieben ist in diesem Zusammenhang, beispielsweise aus der EP 0 523 838 A1, der EP 0 501 577 A1 sowie der EP 0 740 957 A1, bekannt. Letztere können auch aktive Phasen von Katalysatoren bilden. Aluminiumoxidbasierte Katalysatoren werden im Allgemeinen in Anwesenheit von Wasser bei Temperaturen von 200 bis 700 °C und Drücken von 0,1 bis 2 MPa, insbesondere bei Temperaturen von 300 bis 570 °C und Drücken von 0,1 bis 1 MPa, eingesetzt. Weitere Reaktionsbedingungen zur Skelettisomerisierung können den genannten Druckschriften entnommen werden.

Durch die Skelettisomerisierung erhaltene n-C4-Verbindungen werden vorteilhafterweise ebenfalls den zuvor erwähnten milden Spaltbedingungen unterworfen; insbesondere können diese mit dem n-C4-Teilstrom, der erfindungsgemäß aus dem C4-Kohlenwasserstoffstrom gewonnen wird, vereinigt werden. Bei der Skelettisomerisierung (zunächst) nicht umgesetzte iso-C4-Verbindungen können zumindest teilweise aus einer entsprechenden Anlage ausgeschleust, scharfen Spaltbedingungen unterworfen und/oder erneut einem beliebigen der zuvor und nachfolgend erläuterten Prozesse unterworfen werden. Insbesondere kann vorteilhaft sein, zumindest einen Teil dieser iso-C4-Verbindungen erneut der erläuterten Skelettisomerisierung zu unterziehen, bis eine vollständige Umsetzung erfolgt ist. Es kann auch vorteilhaft sein, den gesamten in der Skelettisomerisierung erhaltenen Strom, der n-C4-Verbindungen und (nicht umgesetzte) iso-C4-Verbindungen enthält, mit dem als Ausgangsstrom verwendeten C4-Kohlenwasserstoffstrom zu vereinigen. Damit können die enthaltenen n-C4-Verbindungen mittels derselben Vorrichtung abgetrennt werden, die zur Gewinnung des n-C4-Teilstroms und des iso-C4-Teilstroms ohnehin vorhanden ist. Hierbei kann eine Einspeisung an beliebiger Stelle, d.h. stromauf oder stromab beliebiger vor der Gewinnung des n-C4-Teilstroms und des iso-C4-Teilstroms durchgeführter Prozesse, erfolgen, so dass diese auch auf die bei der Skelettisomerisierung erhaltenen Verbindungen Anwendung finden können.

Die Dampfspaltung von C4-Fraktionen unterschiedlicher Herkunft ist Stand der Technik. Eine zuverlässige Vorhersage des Spaltergebnisses ist mit den vorhandenen Werkzeugen möglich. In der Regel liegen diese als Gemische aus verzweigten und unverzweigten C4-Verbindungen vor. In den zuvor erwähnten Frischeinsätzen umfassen diese überwiegend paraffinische Verbindungen, in Recycleströmen von Dampfspaltprozessen oder in Produkten anderer Verarbeitungsverfahren (z.B. aus Raffinerien) überwiegend olefinische Verbindungen.

Insbesondere bei Spaltung von Naphthas bei milden Spaltbedingungen oder einem hohen Anteil an C4-Frischeinsätzen ist auch der Anteil der aus dem entsprechenden Spaltgas erhaltenen C4-Fraktion groß und insbesondere relativ schwachkonzentriert an 1,3-Butadien und gegebenenfalls anderen Wertprodukten, die aus der C4-Fraktion extrahiert werden sollen. Als Folge ist die Gewinnung von 1,3-Butadien unwirtschaftlich.

Insbesondere beim Dampfspalten von Kohlenwasserstoffen bei ungewöhnlich milden Bedingungen kommt es also, wie erwähnt, zu einer deutlichen Mengenerhöhung einiger Produktfraktionen und einer damit verbundenen Reduktion der Konzentration enthaltener Wertprodukte (Verdünnungseffekt). Die Gewinnung der Wertprodukte wird dadurch erschwert bzw. kostspieliger.

Der Erfindung liegt die Erkenntnis zugrunde, dass verzweigte C4-Verbindungen im Spaltofen strukturbedingt in geringem Umfang zur Bildung von 1,3-Butadien beitragen. Eine relativ hohe Methanbildung aus derartigen Verbindungen ist unvermeidbar, insbesondere dann, wenn die verzweigten C4-Verbindungen bis zur vollständigen Umsetzung recycelt werden. Werden also C4-Kohlenwasserstoffströme mit verzweigten und unverzweigten C4-Verbinungen insgesamt unter milden oder gar sehr milden Bedingungen gespalten, resultieren daraus C4-Fraktionen relativ hoher Mengenströme bei gleichzeitig niedriger Konzentration an 1,3-Butadien.

Diesem Effekt wird erfindungsgemäß begegnet, indem aus dem C4-Kohlenwasserstoffstrom vor der Dampfspaltung, beispielsweise destillativ, der n-C4-Teilstrom gewonnen und dieser, bzw. die überwiegend in diesem enthaltenen n-C4-Verbindungen, vorzugsweise jedoch nicht die iso-C4-Verbindungen, bei den angegebenen milden Spaltbedingungen gespalten wird bzw. werden. Die iso-C4-Verbindungen die, wie erläutert, in einem iso-C4-Teilstrom abgetrennt werden und auch nach etwaigen nachgeschalteten Verfahren, z.B. der Skelettisomerisierung, noch als solche vorliegen, können zumindest teilweise als Produkte gewonnen werden.

Vorteilhafterweise wird jedoch der iso-C4-Teilstrom oder ein hiervon abgeleiteter Strom, beispielsweise ein stromab eines Hydrierverfahrens (siehe unten) oder eines Skelettisomerisierungsverfahrens vorliegender Strom oder Teilstrom, zumindest zum Teil bei hoher Spaltschärfe gespalten. In einem solchen Fall werden im Gegensatz zum n-C4-Teilstrom, der insbesondere besonders mild gespalten wird, sehr scharfe Spaltbedingungen verwendet.

Vorteilhafterweise wird also der iso-C4-Teilstrom oder ein hiervon abgeleiteter Strom zumindest teilweise bei einer Spaltschärfe gespalten, bei der in diesem enthaltenes iso-Butan zu mehr als 91% umgesetzt wird. Das "Spalten" umfasst dabei auch hier, den iso-C4-Teilstrom oder den hiervon abgeleiteten Strom (oder einen entsprechenden Anteil) alleine oder zusammen mit weiteren Strömen, gegebenenfalls auch nach voriger Vereinigung zu einem Sammelstrom, in einen Spaltofen gemäß der eingangs getroffenen Definition einzuspeisen und dem Spaltofen ein Spaltgas zu entnehmen.

Die für das Spalten des iso-C4-Teilstroms verwendeten Spaltschärfen sind damit höher als die für das Spalten des n-C4-Teilstroms verwendeten Spaltschärfen, wobei sich die Begriffe "höher" und "geringer" hier jeweils aufeinander beziehen. Vorteilhafterweise wird also zumindest ein Teil des iso-C4-Teilstroms oder eines hiervon abgeleiteten Stroms bei einer ersten Spaltschärfe und zumindest ein Teil des n-C4-Teilstroms oder eines hiervon abgeleiteten Stroms bei einer zweiten Spaltschärfe gespalten, wobei die erste Spaltschärfe höher als die zweite bzw. die zweite geringer als die erste ist.

Durch die Erfindung werden unter Beibehaltung ihrer Vorteile die Nachteile der milden Spaltung vermindert bzw. vollständig vermieden, d.h. die Menge der C4-Fraktion wird verringert und dadurch die Konzentration des Zielprodukts, hier insbesondere 1,3-Butadien erhöht. Der spezifische Extraktionsaufwand wird verringert.

Der Kern der Erfindung besteht also in der Minimierung der C4-Fraktion insgesamt durch die selektive Anwendung milder, insbesondere sehr milder Spaltbedingungen auf die n-C4-Verbindungen, so dass die durch diese erzielbaren hohen Selektivitäten, z.B. in Richtung von 1,3-Butadien, erreicht werden. Vorteilhafterweise wird dabei eine gezielte Erhöhung der strukturellen Umsetzung von iso-C4-Verbindungen, beispielsweise durch das scharfe Spalten nach einer zuvor erfolgten Abtrennung der n-C4-Verbindungen, vorgenommen.

Die Erfindung sieht zur Verbesserung der Trennung der iso- und n-C4-Verbindungen ferner vor, in dem C4-Kohlenwasserstoffstrom, also dem Kohlenwasserstoffstrom, der überwiegend verzweigte und unverzweigte Kohlenwasserstoffe mit vier Kohlenstoffatomen aufweist, enthaltenes 1-Buten zumindest teilweise zu 2-Buten umzusetzen. Hierzu kommt ein Hydroisomerisierungsverfahren zum Einsatz.

Der C4-Kohlenwasserstoffstrom oder zumindest ein von dem C4-Kohlenwasserstoffstrom abgeleiteter (d.h. abgetrennter, mit einem weiteren Strom vereinigter usw.) Strom wird dabei in zumindest einen Hydroisomerisierungsreaktor eingespeist. Es wird ein an 2-Buten angereicherter und gleichzeitig an 1-Buten abgereicherter C4-Kohlenwasserstoffstrom erhalten, in dem gegebenenfalls auch weitere Komponenten durch das Hydroisomerisierungsverfahren umgesetzt wurden. Beispielsweise kann hierdurch auch eine Entfernung von verbliebenen Spuren von Butadien erfolgen. Die durch das Hydroisomerisierungsverfahren nicht umgesetzten Komponenten sind jedoch weiter in diesem Strom enthalten. Ein solcher Strom wird auch als "Abstrom" des Hydroisomerisierungsverfahrens (bzw. eines hierbei verwendeten Hydroisomerisierungsreaktors) bezeichnet. Es handelt sich um einen von dem C4-Kohlenwasserstoffstrom abgeleiteten Strom.

Es kann auch vorteilhaft sein, zu dem C4-Kohlenwasserstoffstrom, der überwiegend verzweigte und unverzweigte Kohlenwasserstoffe mit jeweils vier Kohlenstoffatomen aufweist, vor oder nach der Hydroisomerisierung oder einem anderen Prozess, wenigstens einen weiteren Strom, insbesondere einen Butine (C4-Acetylene) und/oder Kohlenwasserstoffe mit fünf Kohlenstoffatomen enthaltenden Strom zuzuspeisen. Beispielsweise können hierbei bei einer Butadienextraktion (siehe unten) koextrahierte C5-Verbindungen verwendet werden, die damit einer sinnvollen Nutzung zugeführt werden können.

Diese Hydroisomerisierung sorgt für eine Umsetzung des 1-Butens zu 2-Buten in einem entsprechenden C4-Kohlenwasserstoffstrom, wodurch eine Trennung der iso-C4- von den n-C4-Verbindungen deutlich vereinfacht wird. Dies ergibt sich aus dem deutlich höheren Siedepunkt des 2-Butens bzw. seiner beiden Isomere (cis-2-Buten: 3,72 °C bei Atmosphärendruck; trans-2-Buten: 0,88 °C bei Atmosphärendruck) gegenüber dem 1-Buten (-6,26 °C bei Atmosphärendruck). 1-Buten lässt sich hingegen auf Grundlage seines Siedepunkts in der Praxis destillativ nicht von iso-Buten mit nahezu identischem Siedepunkt (-6,9 °C bei Atmosphärendruck) trennen. Der Grundgedanke der vorliegenden Erfindung, nämlich die für sehr milde Spaltbedingungen unvorteilhaften iso-C4-Verbindungen vor der milden Spaltung abzutrennen, lässt sich daher durch die Hydroisomerisierung einfacher und kostengünstiger realisieren. Zusätzlich können durch die Hydroisomerisierung C4-Acetylene zu n-Butenen umgesetzt werden.

Hydroisomerisierungsverfahren an sich sind bekannt und beispielsweise in der EP 1 871 730 B1, der US 2002/169346 A1, der US 6 420 619 B1, der US 6 075 173 A und der WO 93/21137 A1 beschrieben. Bei solchen Verfahren wird typischerweise ein entsprechender Strom in Anwesenheit eines Hydroisomerisierungskatalysators durch einen Hydroisomerisierungsreaktor geführt. Der Hydroisomerisierungsreaktor ist typischerweise als Festbettreaktor ausgebildet. Vorzugsweise wird mittels des Hydroisomerisierungsverfahrens eine weitestgehende Umsetzung des 1-Butens zu 2-Buten erzielt. Die tatsächlich vorgenommene Umsetzung ergibt sich jedoch unter anderem aus Wirtschaftlichkeitserwägungen.

Besondere Vorteile ergeben sich, wenn der iso-C4-Teilstrom, also jener, der überwiegend verzweigte Kohlenwasserstoffe mit vier Kohlenstoffatomen aufweist, oder ein hiervon abgeleiteter Strom, gegebenenfalls vor einem anschließenden Dampfspalten, zumindest teilweise einem Hydrierverfahren unterzogen wird. Hierbei wird das enthaltene iso-Buten (olefinisch) zumindest teilweise zu iso-Butan (paraffinisch) umgesetzt. Das iso-Butan lässt sich im nachgeschalteten Spaltverfahren, d.h. bei der höheren Spaltschärfe, leichter bzw. zu besser verwertbaren Produkten umsetzen. Dies ermöglicht eine weitere Verringerung der Menge der C4-Fraktion und dadurch eine Konzentration der Zielprodukte, wie oben erwähnt.

Zur Hydrierung und Hydroisomerisierung von Olefinen oder olefinhaltigen Kohlenwasserstoffgemischen sind aus dem Stand der Technik zahlreiche katalytische Verfahren bekannt, die auch im Rahmen der vorliegenden Erfindung zum Einsatz kommen können. Hydrierkatalysatoren besitzen als hydrieraktive Komponente ein oder mehrere Elemente der 6., 7. oder 8. Nebengruppe des Periodensystems in elementarer oder gebundener Form. Als Hydroisomerisierungskatalysatoren werden typischerweise Edelmetalle der 8. Nebengruppe in elementarer Form eingesetzt. Sie können mit unterschiedlichen Zusätzen dotiert sein, um bestimmte Katalysatoreigenschaften, beispielsweise Lebensdauer, Resistenz gegen bestimmte Katalysatorgifte, Selektivität oder Regenerierbarkeit zu beeinflussen. Die Hydrier- und Hydroisomerisierungskatalysatoren enthalten die aktive Komponente vielfach auf Trägern, beispielsweise Mordeniten, Zeolithen, Al2O3-Modifikationen, SiO2-Modifikationen und anderen.

Zur weitgehenden Hydrierung der Olefine werden im Allgemeinen Reaktionstemperaturen von 150 bis 250 °C angewandt, die Hydroisomerisierung erfolgt bei deutlich geringeren Temperaturen. Das thermodynamische Gleichgewicht liegt bei diesen niedrigeren Temperaturen auf Seiten der internen Olefine, hier 2-Buten.

Die zuvor erläuterten Verfahrensvarianten können umfassen, den erwähnten C4-Kohlenwasserstoffstrom zumindest teilweise aus wenigstens einem Spaltgasstrom zu bilden, der beim erfindungsgemäßen Dampfspalten des n-C4-Teilstroms oder entsprechender Anteile und/oder abgeleiteter Ströme hiervon, ggf. zusammen mit Frischeinsatz, erzeugt wird.

Der C4-Kohlenwasserstoffstrom kann jedoch auch zumindest teilweise aus einem Spaltgas, das durch Dampfspalten eines Frischeinsatzes erhalten wird, oder aus einem ungespaltenen Frischeinsatz gebildet werden. Diese Varianten ermöglichen eine sehr flexible Einstellung der jeweils erwünschten Gehalte des C4-Kohlenwasserstoffstroms hinsichtlich der einzelnen C4-Verbindungen.

Die Dampfspaltung erfolgt im Rahmen der vorliegenden Erfindung vorteilhafterweise unter Verwendung einer Dampfmenge von 0,4 kg/kg, insbesondere von 0,2 bis 0,7 kg/kg, beispielsweise von 0,3 bis 0,5 kg/kg mit gleichen oder unterschiedlichen Werten in den jeweils verwendeten Spaltöfen. Entsprechende Werte können insbesondere auch an weitere gespaltene Einsätze angepasst werden.

Werden unterschiedliche Spaltschärfen verwendet, können diese vorteilhafterweise jeweils in wenigstens einem Spaltofen eingestellt werden, dem zumindest ein weiterer Ofeneinsatz zugeführt wird. Beispielsweise kann ein für einen entsprechenden Durchsatz ausgelegter Spaltofen verwendet werden, der bei geringer Spaltschärfe betrieben wird und in dem neben dem n-C4-Strom auch ein "regulärer" Frischeinsatz mild gespalten wird. Ist eine entsprechende Verfahrensvariante realisiert, kann der iso-C4-Strom auch alleine in einem bei der höheren Spaltschärfe betriebenen Spaltofen gespalten werden. In bestimmten Fällen, beispielsweise wenn aus Kostengründen gleichartige Spaltöfen eingesetzt werden, kann es jedoch sinnvoller sein, den iso-C4-Strom zusammen mit einem Frischeinsatz scharf zu spalten.

Es versteht sich, dass nicht der gesamte iso-C4-Strom, falls gebildet, scharf gespalten werden muss. Es kann auch vorgesehen sein, einen Teil des iso-C4-Stroms mild zu spalten. Zumindest ein Teil wird jedoch scharf gespalten, wodurch eine entsprechende Mengenreduktion, wie zuvor erläutert, erzielt wird.

Wie erwähnt, hat die vorliegende Erfindung insbesondere den Zweck, ein Verfahren zu verbessern, bei dem 1,3-Butadien aus dem Kohlenwasserstoffstrom abgetrennt wird. Hierbei eignen sich sämtliche bekannten Verfahren zur Extraktion von 1,3-Butadien. Weitere Vorteile können sich ergeben, wenn in dem Kohlenwasserstoffstrom enthaltenes Isobuten nach der Abtrennung des 1,3-Butadiens zumindest teilweise zu einem tert-Butylether umgesetzt und dieser ebenfalls, beispielsweise vor einer Hydroisomerisierung, extrahiert wird. Die Herstellung von Methyl-tert-butylether (2-Methoxy-2-methylpropan, MTBE) ist grundsätzlich bekannt. MTBE wird großtechnisch säurekatalytisiert aus Isobuten und Methanol hergestellt, das dem Kohlenwasserstoffstrom zugegeben wird. MTBE wird hauptsächlich als Klopfschutzmittel verwendet, findet aber auch zunehmend Verwendung als Lösungsmittel sowie Extraktionsmittel in der organischen Chemie. Ethanol ergibt Ethyl-tert-butylether. Auch andere Alkohole können eingesetzt werden.

Die wenigstens eine Trenneinrichtung umfasst vorteilhafterweise zumindest eine Trennsäule und die Dampfspalteinrichtung umfasst vorteilhafterweise zumindest zwei Spaltöfen, die für einen Betrieb bei unterschiedlichen Spaltschärfen eingerichtet sind.

Die Erfindung wird nachfolgend unter Bezugnahme auf die beigefügten Figuren gegenüber dem Stand der Technik erläutert.

### Kurze Beschreibung der Zeichnungen

Figur 1A veranschaulicht schematisch den Ablauf eines Verfahrens zur Erzeugung von Kohlenwasserstoffen gemäß dem Stand der Technik.
Figur 1B veranschaulicht schematisch den Ablauf eines Verfahrens zur Erzeugung von Kohlenwasserstoffen gemäß dem Stand der Technik.
Figur 2 veranschaulicht schematisch den Ablauf eines Verfahrens zur Erzeugung von Kohlenwasserstoffen gemäß einer Ausführungsform der Erfindung.
Figur 3 veranschaulicht schematisch den Ablauf eines Verfahrens zur Erzeugung von Kohlenwasserstoffen gemäß einer Ausführungsform der Erfindung.
Figur 4 veranschaulicht schematisch den Ablauf eines Verfahrens zur Erzeugung von Kohlenwasserstoffen gemäß einer Ausführungsform der Erfindung.
Figur 5 veranschaulicht schematisch den Ablauf eines Verfahrens zur Erzeugung von Kohlenwasserstoffen gemäß einer Ausführungsform der Erfindung.
Figur 6 veranschaulicht schematisch den Ablauf eines Verfahrens zur Erzeugung von Kohlenwasserstoffen gemäß einer Ausführungsform der Erfindung.

In den Figuren sind einander entsprechende Elemente mit identischen Bezugszeichen versehen und werden der Übersichtlichkeit halber nicht wiederholt erläutert.

### Ausführliche Beschreibung der Zeichnungen

In Figur 1A ist der Ablauf eines Verfahrens zur Erzeugung von Kohlenwasserstoffen gemäß dem Stand der Technik in Form eines schematischen Ablaufplans dargestellt. Kern des Verfahrens ist hier ein Dampfspaltprozess 10, der unter Verwendung eines oder mehrerer Spaltöfen 11 bis 13 durchgeführt werden kann. Nachfolgend wird nur der Betrieb des Spaltofens 11 erläutert, die weiteren Spaltöfen 12 und 13 können in entsprechender Weise arbeiten.

Der Spaltofen 11 wird mit einem Strom A als Ofeneinsatz beschickt, bei dem es sich zumindest teilweise um einen sogenannten Frischeinsatz handeln kann, der aus anlagenexternen Quellen zur Verfügung gestellt wird, und zu einem Teil um einen sogenannten Recyclestrom, der in dem Verfahren selbst gewonnen wird, wie unten erläutert. Auch die anderen Spaltöfen 12 und 13 können mit entsprechenden Strömen beschickt werden. Unterschiedliche Ströme können auch in unterschiedliche Spaltöfen 11 bis 13 eingespeist werden, ein Strom kann auf mehrere Spaltöfen aufgeteilt werden oder mehrere Teilströme können zu einem Sammelstrom vereinigt werden, der beispielsweise als Strom A einem der Spaltöfen 11 bis 13 zugeführt wird.

Durch Dampfspalten in dem Dampfspaltprozess 10 wird ein Rohgasstrom B erhalten, der bisweilen bereits an dieser Stelle als Spaltgasstrom bezeichnet wird. Der Rohgasstrom B wird in einer Reihe nicht dargestellter Aufbereitungsstufen eines Aufbereitungsprozesses 20 aufbereitet, beispielsweise einem sogenannten Ölquench unterzogen, vorfraktioniert, verdichtet, weiter gekühlt und getrocknet.

Der entsprechend behandelte Strom B, das eigentliche Spaltgas C, wird anschließend einem Trennprozess 30 unterworfen. In diesem wird eine Anzahl von Fraktionen gewonnen, die hier, wie eingangs erläutert, entsprechend der Kohlenstoffanzahl der überwiegend enthaltenen Kohlenwasserstoffe bezeichnet werden. Der in der Figur 1A dargestellte Trennprozess 30 arbeitet nach dem Prinzip "Demethanizer First", ein Trennprozess nach dem Prinzip "Deethanizer First" ist in Figur 1B dargestellt.

In dem Trennprozess 30 wird aus dem Spaltgas C zunächst in einer ersten Trenneinheit 31 (dem sogenannten Demethanizer) eine C1- bzw. C1 minus-Fraktion (mit dem Bezugszeichen C1 bezeichnet) gasförmig abgetrennt, die, falls nicht bereits zuvor entfernt, auch noch Wasserstoff enthalten kann. Sie wird typischerweise als Brenngas verwendet. Es verbleibt eine flüssige C2plus-Fraktion (Bezugszeichen C2+), die in eine zweite Trenneinheit 32 (den sogenannten Deethanizer) überführt wird.

In der zweiten Trenneinheit 32 wird aus der C2plus-Fraktion eine C2-Fraktion (Bezugszeichen C2) gasförmig abgetrennt und beispielsweise einem Hydrotreatmentprozess 41 unterzogen, um enthaltenes Acetylen zu Ethylen umzusetzen. Anschließend wird die C2-Fraktion in einer C2-Trenneinheit 35 in Ethylen (Bezugszeichen C2H4) und Ethan (Bezugszeichen C2H6) aufgetrennt. Letzteres kann als Recyclestrom D in einem oder mehreren Spaltöfen 11 bis 13 erneut dem Dampfspaltprozess 10 unterworfen werden. Im dargestellten Beispiel werden die Recycleströme D und E zu dem Strom A zugegeben. Die Recycleströme D und E und der Strom A können auch in unterschiedliche Spaltöfen 11 bis 13 geführt werden.

In der zweiten Trenneinheit 32 verbleibt eine flüssige C3plus-Fraktion (Bezugszeichen C3+), die in eine dritte Trenneinheit 33 (den sogenannten Depropanizer) überführt wird. In der dritten Trenneinheit 33 wird aus der C3plus-Fraktion eine C3-Fraktion (Bezugszeichen C3) abgetrennt und einem weiteren Hydrotreatmentprozess 42 unterzogen, um in der C3-Fraktion enthaltenes Propylen zu Propen umzusetzen. Anschließend wird die C3-Fraktion in einer C3-Trenneinheit 36 in Propen (Bezugszeichen C3H6) und Propan (Bezugszeichen C3H8) aufgetrennt. Letzteres kann als Recyclestrom E in einem oder mehreren Spaltöfen 11 bis 13, separat oder mit anderen Strömen, erneut dem Dampfspaltprozess 10 unterworfen werden.

In der dritten Trenneinheit 33 verbleibt eine flüssige C4plus-Fraktion (Bezugszeichen C4+), die in eine vierte Trenneinheit 34 (den sogenannten Debutanizer) überführt wird. In der vierten Trenneinheit 34 wird aus der C4plus-Fraktion eine C4-Fraktion (Bezugszeichen C4, hier als C4-Kohlenwasserstoffstrom bezeichnet) abgetrennt. Es verbleibt eine flüssige C5plus-Fraktion (Bezugszeichen C5+).

Es versteht sich, dass sämtliche der dargestellten Fraktionen auch geeigneten Nachbehandlungsschritten unterworfen werden können. Beispielsweise kann aus dem C4-Kohlenwasserstoffstrom, wie auch unten dargestellt, 1,3-Butadien abgetrennt werden. Es können ferner zusätzliche Recycleströme verwendet werden, die analog zu den Recycleströmen D und E dem Dampfspaltprozess 10 unterworfen werden können.

In Figur 1B ist der Ablauf eines alternativen Verfahrens zur Erzeugung von Kohlenwasserstoffen durch Dampfspalten gemäß dem Stand der Technik in Form eines schematischen Ablaufplans dargestellt. Auch hier ist Kern des Verfahrens ein Dampfspaltprozess 10, der unter Verwendung eines oder mehrerer Spaltöfen 11 bis 13 ablaufen kann. Im Gegensatz zu dem in Figur 1A veranschaulichten Verfahren wird hier das Spaltgas C einem alternativen Trennprozess 30 nach dem Prinzip "Deethanizer First" unterworfen.

In dem Trennprozess 30 wird dabei aus dem Spaltgas C zunächst in einer ersten Trenneinheit 37 eine C2minus-Fraktion (Bezugszeichen C2-) gasförmig abgetrennt, die überwiegend Methan, Ethan, Ethylen und Acetylen und, falls nicht bereits zuvor entfernt, auch noch Wasserstoff enthalten kann. Die C2minus-Fraktion wird insgesamt einem Hydrotreatmentprozess 43 unterzogen, um enthaltenes Acetylen zu Ethylen umzusetzen. Anschließend wird aus der C2minus-Fraktion in einer C2minus-Trenneinheit 38 eine C1-Fraktion abgetrennt und wie oben weiter verwendet. Es verbleibt eine C2-Fraktion, die in einer C2-Trenneinheit 35 wie oben in Ethylen und Ethan aufgetrennt wird. Letzteres kann auch hier als Recyclestrom D in einem oder mehreren Spaltöfen 11 bis 13 erneut dem Dampfspaltprozess 10 unterworfen werden. In der ersten Trenneinheit 37 verbleibt eine flüssige C3plus-Fraktion, die in den Trenneinheiten 33 bis 36 und der Hydrotreatmenteinheit 42, wie zu Figur 1 erläutert, behandelt wird.

Dem Fachmann sind, beispielsweise aus dem eingangs erwähnten Artikel "Ethylene" in Ullmann's Encyclopedia of Industrial Chemistry, mehrere weitere Verfahrensalternativen bekannt, die sich insbesondere in der Aufbereitung des Spaltgases C und/oder dem verwendeten Trennprozess unterscheiden.

Auch der C4-Kohlenwasserstoffstrom kann in Teilen als entsprechender Recyclestrom in einem oder mehreren der Spaltöfen 11 bis 13 erneut dem Dampfspaltprozess 10 unterworfen werden. Insbesondere dann, wenn hierbei milde Spaltbedingungen verwendet werden, können in dem C4-Kohlenwasserstoffstrom enthaltene verzweigte C4-Verbindungen (iso-C4-Verbindungen) jedoch in geringerem Umfang als n-C4-Verbindungen umgesetzt werden und finden sich daher zu einem großen Teil erneut im Spaltgasstrom C wieder. Die iso-C4-Verbindungen werden daher mehrfach durch eine entsprechende Anlage zirkuliert. Die Folge einer derartigen milden Dampfspaltung ist damit eine deutliche Mengenerhöhung einiger Produktfraktionen, hier der iso-C4-Verbindungen, und eine damit verbundene Reduktion der Konzentration enthaltener Wertprodukte, hier beispielsweise von 1,3-Butadien, durch entsprechende Verdünnungseffekte. Die Gewinnung der Wertprodukte wird dadurch erschwert bzw. kostspieliger. Die iso-C4-Verbindungen tragen, mit anderen Worten, strukturbedingt praktisch nicht zur 1,3-Butadienbildung bei. Die Bildung einer relativ hohen Menge weitgehend wertlosen Methans ist unvermeidbar, insbesondere dann, wenn die iso-C4-Verbindungen bis zur vollständigen Umsetzung recycelt werden.

Werden also C4-Kohlenwasserstoffströme mit iso-C4-Verbindungen, gleich welcher Herkunft, unter milden oder sehr milden Bedingungen gespalten, resultieren hieraus wiederum C4-Produktfraktionen relativ hoher Mengenströme bei gleichzeitig niedriger 1,3-Butadienkonzentration.

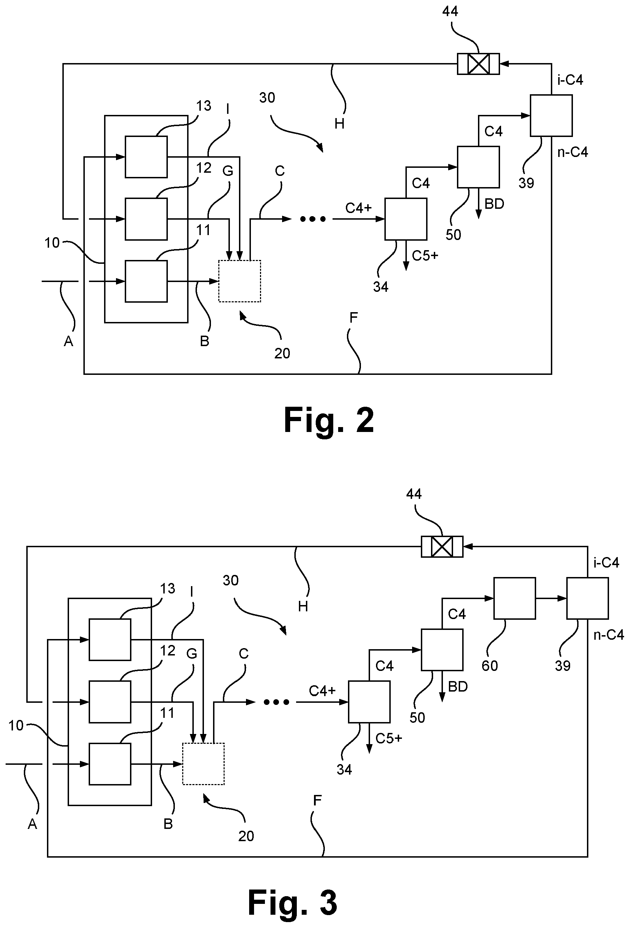

In Figur 2 ist der Ablauf eines Verfahrens zur Erzeugung von Kohlenwasserstoffen durch Dampfspalten gemäß einer Ausführungsform der Erfindung in Form eines schematischen Ablaufplans dargestellt. Auch hier ist Kern des Verfahrens ein Dampfspaltprozess 10, der unter Verwendung von Spaltöfen 11 bis 13 ablaufen kann. Zur Veranschaulichung der universellen Einsetzbarkeit des hier dargestellten Verfahrens ist die Gewinnung einer C4plus-Fraktion aus dem Spaltgas C nicht dargestellt, diese kann jedoch wie in den Figuren 1A und 1B oder auf jede andere in der Fachwelt bekannte Weise ablaufen. Die C4plus-Fraktion wird in dem hier dargestellten Beispiel einer Trenneinheit 34 zugeführt, die wie oben beschrieben arbeitet. Falls in einem Dampfspaltverfahren jedoch keine oder nur wenige C5plus-Kohlenwasserstoffe gebildet werden, könnte auf die Verwendung dieser Trenneinheit 34 auch verzichtet werden. Ein C4-Kohlenwasserstoffstrom kann jedoch auch von anlagenextern, z.B. aus einer Raffinerie, bereitgestellt werden.

Ein beispielsweise aus der Trenneinheit 34 erhaltener C4-Kohlenwasserstoffstrom kann einer 1,3-Butadiengewinnungseinheit 50 zugeführt werden, in der 1,3-Butadien, hier mit BD bezeichnet, extrahiert wird. 1,3-Butadien stellt hier eines der erwünschten Wertprodukte dar, die verbleibenden Anteile des C4-Kohlenwasserstoffstroms sind zu einem überwiegenden Anteil von geringerem wirtschaftlichem Wert und "verdünnen" das erwünschte 1,3-Butadien, was dessen Extraktion erschwert.

Die Erfindung sieht gemäß der dargestellten Ausführungsform vor, in einer Trenneinheit 39 iso-C4- und n-C4-Verbindungen (Bezugszeichen i-C4 und n-C4), also verzweigte und unverzweigte C4-Verbindungen, voneinander zu trennen und entsprechende Teilströme zu gewinnen. Der Teilstrom, der überwiegend die iso-C4-Verbindungen enthält, wird hier als iso-C4-Teilstrom bezeichnet. Dieser kann als Recyclestrom H zurückgeführt und entweder erneut dem Dampfspaltprozess 10 oder einem separat zu dem Dampfspaltprozess 10 implementierten weiteren Dampfspaltprozess unterworfen werden. Vorzugsweise wird der iso-C4-Teilstrom dabei scharfen Spaltbedingungen unterworfen, wofür hier der Spaltofen 12 eingerichtet sei. Zuvor kann eine Hydrierung von iso-Buten vorgenommen werden, wie mit Block 44 veranschaulicht. Ein dem Spaltofen 12 entnommener Strom G kann beispielsweise zu dem Spaltgas C zugegeben werden, gegebenenfalls nachdem dieser zuvor ebenfalls dem Aufbereitungsprozess 20 unterworfen wurde.

Der Teilstrom, der überwiegend die n-C4-Verbindungen enthält und hier als n-C4-Teilstrom bezeichnet wird, kann als Recyclestrom F zurückgeführt und auch hier entweder erneut dem Dampfspaltprozess 10 oder auch hier einem separat zu dem Dampfspaltprozess 10 implementierten weiteren Dampfspaltprozess unterworfen werden. Vorzugsweise werden die n-C4-Verbindungen dabei milden bis sehr milden Spaltbedingungen unterworfen, wofür hier der Spaltofen 13 eingerichtet sei. Ein dem Spaltofen 13 entnommener Strom I kann beispielsweise zu dem Spaltgas C zugegeben werden, gegebenenfalls nachdem dieser zuvor dem Aufbereitungsprozess 20 unterworfen wurde.

In Figur 3 ist der Ablauf eines Verfahrens zur Erzeugung von Kohlenwasserstoffen durch Dampfspalten gemäß einer weiteren Ausführungsform der Erfindung in Form eines schematischen Ablaufplans dargestellt. Wiederum ist Kern des Verfahrens ein Dampfspaltprozess 10, der unter Verwendung von Spaltöfen 11 bis 13 ablaufen kann. Auch hier ist zur Veranschaulichung der universellen Einsetzbarkeit des dargestellten Verfahrens die Gewinnung einer C4plus-Fraktion aus dem Spaltgas C nicht dargestellt, diese kann jedoch wie in den Figuren 1A und 1B oder auf jede andere in der Fachwelt bekannte Weise ablaufen. Die C4plus-Fraktion wird auch in dem hier dargestellten Beispiel einer Trenneinheit 34 zugeführt, die wie oben beschrieben arbeitet. Wiederum könnte, falls in einem Dampfspaltverfahren keine oder nur wenige C5plus-Kohlenwasserstoffe gebildet werden, auf die Verwendung dieser Trenneinheit 34 verzichtet werden. Ein C4- Kohlenwasserstoffstrom kann auch hier wiederum von anlagenextern, z.B. aus einer Raffinerie, bereitgestellt werden.

Ein beispielsweise aus der Trenneinheit 34 erhaltener C4-Kohlenwasserstoffstrom kann einer 1,3-Butadiengewinnungseinheit 50 zugeführt werden, in der 1,3-Butadien, hier mit BD bezeichnet, extrahiert wird. 1,3-Butadien stellt hier eines der erwünschten Wertprodukte dar, die verbleibenden Anteile des C4-Kohlenwasserstoffstroms sind zu einem überwiegenden Anteil von geringerem wirtschaftlichem Wert und "verdünnen" das erwünschte 1,3-Butadien, was dessen Extraktion erschwert.

Die Erfindung sieht gemäß der dargestellten Ausführungsform vor, den C4-Kohlenwasserstoffstrom stromab der Butadiengewinnungseinheit 50, hier weiter mit dem Bezugszeichen C4 bezeichnet, einem Hydroisomerisierungsreaktor 60 zuzuführen und in diesem 1-Buten zumindest teilweise zu 2-Buten umzusetzen.

Anschließend ist auch hier vorgesehen, in einer Trenneinheit 39 iso-C4- und n-C4-Verbindungen voneinander zu trennen und entsprechende Teilströme (n-C4-Teilstrom und iso-C4-Teilstrom) zu gewinnen. Die Erfindung kann auch lediglich die Gewinnung des n-C4-Teilstroms umfassen, der iso-C4-Teilstrom oder in diesem enthaltene Verbindungen können aus der Anlage ausgeleitet werden.

Der iso-C4-Teilstrom kann als Recyclestrom H zurückgeführt und entweder erneut dem Dampfspaltprozess 10 oder einem separat zu diesem implementierten weiteren Dampfspaltprozess unterworfen werden, wie oben erläutert. Auch eine Hydrierung von iso-Buten kann vorgenommen werden, wie mit Block 44 veranschaulicht. Der n-C4-Teilstrom kann auch hier als Recyclestrom F zurückgeführt und entweder erneut dem Dampfspaltprozess 10 oder auch hier einem separat zu dem Dampfspaltprozess 10 implementierten weiteren Dampfspaltprozess unterworfen werden, wie erläutert.

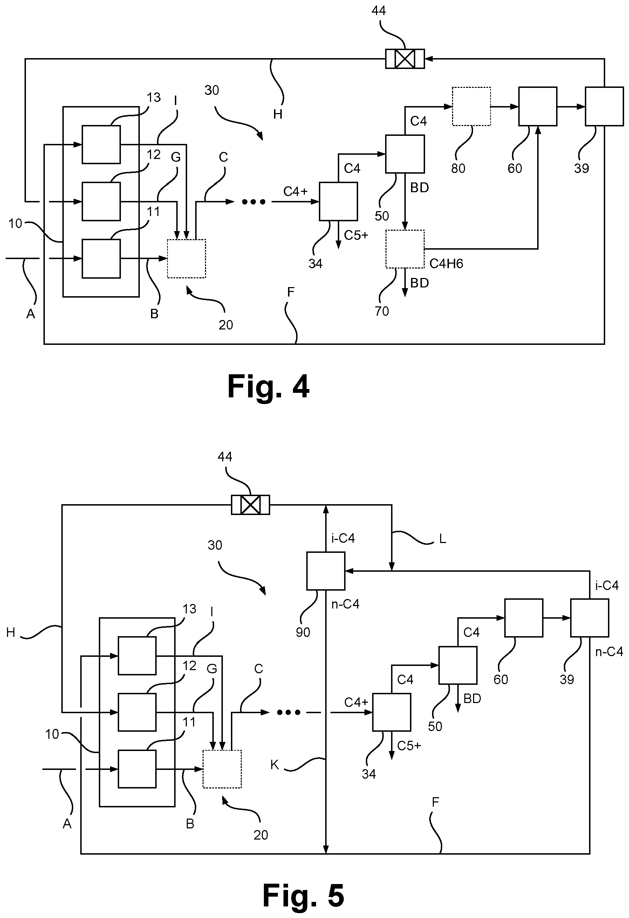

In Figur 4 ist der Ablauf eines Verfahrens zur Erzeugung von Kohlenwasserstoffen durch Dampfspalten gemäß einer weiteren Ausführungsform der Erfindung in Form eines schematischen Ablaufplans dargestellt.

Gegenüber Figur 3 ist hier eine zusätzliche Einheit 70 explizit dargestellt, die dafür eingerichtet ist, bei der Extraktion von Butadien in der Butadiengewinnungseinheit 50 unerwünschterweise koextrahierte Komponenten, beispielsweise C4-Acetylene (Bezugszeichen C4H6), abzutrennen und ebenfalls dem Hydroisomerisierungsreaktor 60 zuzuführen. Diese Einheit 70 ist in der praktischen Realisierung insbesondere in der Butadiengewinnungseinheit 50 integriert und kann auch als Teil der in Figur 3 dargestellten Butadiengewinnungseinheit 50 vorgesehen sein. Alternativ oder zusätzlich dazu kann eine Einrichtung 80 vorgesehen sein, die iso-Buten in dem C4-Kohlenwasserstoffstrom nach der Abtrennung des 1,3-Butadiens zumindest teilweise zu Methyl-tert-butylether umsetzt und den Methyl-tert-butylether ebenfalls aus dem Kohlenwasserstoffstrom abtrennt (nicht dargestellt). Alternativ ist auch beispielsweise die Bildung von Ethyl-tert-butylether möglich.

In Figur 5 ist der Ablauf eines Verfahrens zur Erzeugung von Kohlenwasserstoffen durch Dampfspalten gemäß einer weiteren Ausführungsform der Erfindung in Form eines schematischen Ablaufplans dargestellt. Wiederum ist Kern des Verfahrens ein Dampfspaltprozess 10, der unter Verwendung von Spaltöfen 11 bis 13 ablaufen kann. Zur weiteren Verfahrensführung und den hier verwendeten Einrichtungen sei auf die Erläuterungen zu den Figuren 2 bis 4 verwiesen.

Der iso-C4-Teilstrom aus der Trenneinheit 39 wird hier einem Skelettisomerisierungsreaktor 90 zugeführt, der dafür eingerichtet ist, zumindest einen Teil der in dem iso-C4-Teilstrom enthaltenen iso-C4-Verbindungen zu den entsprechenden n-C4-Verbindungen umzusetzen. Hierbei, oder in einem nachgeschalteten Trennschritt, werden erneut ein Teilstrom mit überwiegend (nicht umgesetzten) iso-C4-Verbindungen (Bezugszeichen i-C4) und ein Teilstrom mit überwiegend n-C4-Verbindungen erhalten. Letzterer kann beispielsweise als Strom K mit dem Strom F, der die in der Trenneinheit 39 erhaltenen n-C4-Verbindungen enthält, vereinigt und dem Dampfspaltprozess 10 unterworfen werden, wobei, beispielsweise in Spaltofen 13, milde Spaltbedingungen zum Einsatz kommen. Die bei der Skelettisomerisierung nicht umgesetzten iso-C4-Verbindungen können, wie mit Strom L veranschaulicht, auch teilweise oder vollständig in den Skelettisomerisierungsreaktor 90 zurückgeführt werden, um sukzessive eine weitgehende oder vollständige Umsetzung zu erzielen. Es kann jedoch auch vorgesehen sein, einen hieraus gebildeten Strom H, gegebenenfalls nach Hydrierung gemäß Block 44, zu spalten (beispielsweise bei scharfen Spaltbedingungen im Spaltofen 12).

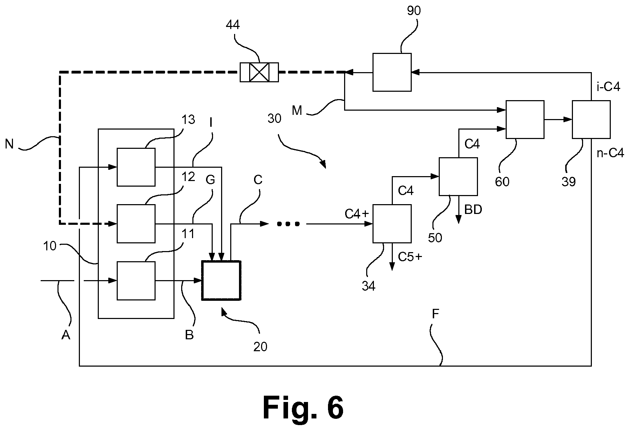

In Figur 6 ist der Ablauf eines Verfahrens zur Erzeugung von Kohlenwasserstoffen durch Dampfspalten gemäß einer weiteren Ausführungsform der Erfindung in Form eines schematischen Ablaufplans dargestellt.

Gegenüber Figur 5 wird hier dem Skelettisomerisierungsreaktor 90 ein noch nicht in n- und iso-C4-Verbindungen aufgetrennter Strom entnommen. Zumindest ein Teil hiervon kann als Strom M über den Hydroisomerisierungsreaktor 60 in die Trenneinheit 39 zurückgeführt werden, wodurch letztlich erreicht wird, dass die in dem Skelettisomerisierungsreaktor 90 nicht umgesetzten iso-C4-Verbindungen im Kreis geführt werden, um sukzessive eine weitgehende oder vollständige Umsetzung zu erzielen. Bei Bedarf kann jedoch auch ein hier gestrichelt dargestellter Strom N gegebenenfalls nach Hydrierung gemäß Block 44, gespalten werden (beispielsweise bei scharfen Spaltbedingungen in dem Spaltofen 12).

Wenngleich nicht dargestellt, versteht sich, dass den Spaltöfen 11 bis 13 noch weitere Recycleströme oder Frischeinsätze zugeführt werden können.

In den zuvor erläuterten Figuren sind jeweils Teilaspekte dargestellt, die jeweils auch in anderer Kombination miteinander zum Einsatz kommen können.

## Patentansprüche

1. Verfahren zur Erzeugung von Kohlenwasserstoffprodukten, das umfasst:
a) Bereitstellen eines C4-Kohlenwasserstoffstroms (C4), der mindestens 80% auf Gewichtsbasis verzweigte und unverzweigte Kohlenwasserstoffe mit jeweils vier Kohlenstoffatomen aufweist, und
b) Gewinnen eines n-C4-Teilstroms (n-C4), der mindestens 80 % auf Gewichtsbasis unverzweigte Kohlenwasserstoffe mit vier Kohlenstoffatomen aufweist, und eines iso-C4-Teilstroms (i-C4), der überwiegend verzweigte Kohlenwasserstoffe mit vier Kohlenstoffatomen aufweist, aus dem C4-Kohlenwasserstoffstrom (C4) oder einem hiervon abgeleiteten Strom,
**gekennzeichnet durch**
c) Dampfspalten zumindest eines Teils des n-C4-Teilstroms (n-C4) oder eines hiervon abgeleiteten Stroms bei einer Spaltschärfe, bei der mindestens 50 % und höchstens 92% von hierin enthaltenem n-Butan umgesetzt werden.

2. Verfahren nach Anspruch 1, **gekennzeichnet durch**:
- Umsetzen zumindest eines Teils von in dem iso-C4-Teilstrom (i-C4) enthaltenen verzweigten Kohlenwasserstoffen zu unverzweigten Kohlenwasserstoffen durch Skelettisomerisieren.

3. Verfahren nach Anspruch 1 oder 2, **gekennzeichnet durch**:
- Umsetzen zumindest eines Teils von in dem C4-Kohlenwasserstoffstrom (C4) enthaltenem 1-Buten zu 2-Buten durch Hydroisomerisieren vor dem Gewinnen des n-C4- und des iso-C4-Teilstroms (n-C4, i-C4) gemäß b).

4. Verfahren nach einem der vorstehenden Ansprüche, **gekennzeichnet durch**:
- Dampfspalten zumindest eines Teils des iso-C4-Teifstroms (iso-C4) oder eines hiervon abgeleiteten Stroms bei einer Spaltschärfe, bei der mehr als 91% von hierin enthaltenem iso-Butan umgesetzt werden.

5. Verfahren nach einem der vorstehenden Ansprüche, bei dem beim Dampfspalten gemäß c) eine Spaltschärfe verwendet wird, bei der weniger als 90%, 88%, 86%, 84%, 82%, 80%, 78%, 76%, 74%, 72%, 70% oder 65% und mehr als 50% oder 60% des n-Butans umgesetzt werden.

6. Verfahren nach einem der vorstehenden Ansprüche, bei dem der iso-C4-Teilstrom oder ein hiervon abgeleiteter Strom (i-C4) zumindest teilweise einem Hydrierverfahren unterzogen wird.

7. Verfahren nach einem der vorstehenden Ansprüche, bei dem der gemäß a) bereitgestellte C4-Kohlenwasserstoffstrom (C4) zumindest teilweise aus zumindest einem durch Dampfspalten gemäß c) erhaltenen Spaltgas (C) erzeugt wird.

8. Verfahren nach einem der vorstehenden Ansprüche, bei dem der gemäß a) bereitgestellte C4-Kohlenwasserstoffstrom (C4) zumindest teilweise aus einem Spaltgas (C) erzeugt wird, das durch Dampfspalten eines Frischeinsatzes (A), insbesondere von einer oder mehreren Erdölfraktionen, Erdgaskomponenten mit zwei bis vier Kohlenstoffatomen und/oder Erdgaskondensaten, gebildet wird.

9. Verfahren nach einem der vorstehenden Ansprüche, bei dem der gemäß a) bereitgestellte Kohlenwasserstoffstrom (C4) zumindest teilweise aus einem zuvor nicht einem Dampfspaltverfahren unterworfenen Frischeinsatz (A), insbesondere aus einer oder mehreren Erdölfraktionen, Erdgaskomponenten mit zwei bis vier Kohlenstoffatomen und/oder Erdgaskondensaten und/oder wenigstens einem Produkt eines Raffinerieprozesses, gebildet wird.

10. Verfahren nach einem der vorstehenden Ansprüche, bei dem das Dampfspalten unter Verwendung einer Dampfmenge von 0,4 kg/kg, insbesondere von 0,2 bis 0,7 kg/kg, durchgeführt wird.

11. Verfahren nach einem der vorstehenden Ansprüche, bei dem das Dampfspalten in wenigstens einem Spaltofen (11, 12, 13) durchgeführt wird, dem zumindest ein weiterer Ofeneinsatz (A) in Form wenigstens eines Recyclestroms und/oder wenigstens eines Frischeinsatzes zugeführt wird.

12. Verfahren nach einem der vorstehenden Ansprüche, bei dem 1,3-Butadien (BD) vor dem Gewinnen des n-C4- und des iso-C4-Teilstroms (i-C4, n-C4) gemäß b) aus dem C4-Kohlenwasserstoffstrom (C4) abgetrennt wird.

13. Verfahren nach Anspruch 12, bei dem in dem Kohlenwasserstoffstrom (C4) enthaltenes Isobuten nach der Abtrennung des 1,3-Butadiens (BD) zumindest teilweise zu einem tert-Butylether umgesetzt und der tert-Butylether ebenfalls aus dem Kohlenwasserstoffstrom (C4) abgetrennt wird.

14. Verfahren nach einem der vorstehenden Ansprüche, bei dem zu dem C4-Kohlenwasserstoffstrom (C4) wenigstens ein weiterer Strom, insbesondere ein Butine und/oder Kohlenwasserstoffe mit fünf Kohlenstoffatomen enthaltender Strom, zugespeist wird.

## Claims

1. Process for producing hydrocarbon products, which comprises:
a) providing a C4 hydrocarbon stream (C4) having at least 80%, based on weight, of branched and unbranched hydrocarbons each having four carbon atoms, and
b) obtaining an n-C4 substream (n-C4) having at least 80%, based on weight, of unbranched hydrocarbons having four carbon atoms, and an iso-C4 substream (i-C4) comprising predominantly branched hydrocarbons having four carbon atoms, from the C4 hydrocarbon stream (C4) or a stream derived therefrom,
**characterized by**
c) steamcracking of at least a portion of the n-C4 substream (n-C4) or of a stream derived therefrom at a cracking severity at which at least 50% and at most 92% of n-butane present therein is converted.

2. Process according to Claim 1, **characterized by**:
- conversion of at least a portion of branched hydrocarbons present in the iso-C4 substream (i-C4) to unbranched hydrocarbons by skeletal isomerization.

3. Process according to Claim 1 or 2, **characterized by**:
- conversion of at least a portion of 1-butene present in the C4 hydrocarbon stream (C4) to 2-butene by hydroisomerization prior to the obtaining of the n-C4 and the iso-C4 substreams (n-C4, iso-C4) in b).

4. Process according to any of the preceding claims, **characterized by**:
- steamcracking of at least a portion of the iso-C4 substream (iso-C4) or of a stream derived therefrom at a cracking severity at which more than 91% of iso-butane present therein is converted.

5. Process according to any of the preceding claims, in which the cracking severity used in the steamcracking in c) is one at which less than 90%, 88%, 86%, 84%, 82%, 80%, 78%, 76%, 74%, 72%, 70% or 65% and more than 50% or 60% of the n-butane is converted.

6. Process according to any of the preceding claims, in which the iso-C4 substream or a stream derived therefrom (i-C4) is subjected at least partly to a hydrogenation process.

7. Process according to any of the preceding claims, in which the C4 hydrocarbon stream (C4) provided in a) is at least partly produced from at least one cracking gas (C) obtained by steamcracking in c).

8. Process according to any of the preceding claims, in which the C4 hydrocarbon stream (C4) provided in a) is at least partly produced from a cracking gas (C) which is formed by steamcracking of a fresh feed (A), especially of one or more mineral oil fractions, natural gas components having two to four carbon atoms and/or natural gas condensates.

9. Process according to any of the preceding claims, in which the hydrocarbon stream (C4) provided in a) is at least partly formed from a fresh feed (A) that has not been subjected to a steamcracking process beforehand, especially composed of one or more mineral oil fractions, natural gas components having two to four carbon atoms and/or natural gas condensates and/or at least one product of a refinery process.

10. Process according to any of the preceding claims, in which the steamcracking is conducted using an amount of steam of 0.4 kg/kg, especially of 0.2 to 0.7 kg/kg.

11. Process according to any of the preceding claims, in which the steamcracking is conducted in at least one cracking furnace (11, 12, 13), which is supplied with at least one further furnace feed (A) in the form of at least one recycle stream and/or at least one fresh feed.

12. Process according to any of the preceding claims, in which 1,3-butadiene (BD) is separated from the C4 hydrocarbon stream (C4) prior to the obtaining of the n-C4 and iso-C4 substreams (i-C4, n-C4) in b).

13. Process according to Claim 12, in which isobutene present in the hydrocarbon stream (C4), after the 1,3-butadiene (BD) has been separated off, is at least partly converted to a tert-butyl ether and the tert-butyl ether is likewise separated from the hydrocarbon stream (C4).

14. Process according to any of the preceding claims, in which at least one further stream, especially a stream comprising butynes and/or hydrocarbons having five carbon atoms, is fed into the C4 hydrocarbon stream (C4).

## Revendications

1. Procédé de formation de produits hydrocarbonés, qui comprend :
a) la préparation d'un courant d'hydrocarbures en C4 (C4), qui comprend au moins 80 % en poids d'hydrocarbures ramifiés et non ramifiés contenant chacun quatre atomes de carbone, et
b) l'obtention d'un courant partiel de n-C4 (n-C4), qui comprend au moins 80 % en poids d'hydrocarbures non ramifiés contenant quatre atomes de carbone, et d'un courant partiel d'iso-C4 (i-C4), qui comprend principalement des hydrocarbures ramifiés contenant quatre atomes de carbone, à partir du courant d'hydrocarbures en C4 (C4) ou d'un courant dérivé de celui-ci,
**caractérisé par**
c) le clivage à la vapeur d'au moins une partie du courant partiel de n-C4 (n-C4) ou d'un courant dérivé de celui-ci avec une précision de clivage à laquelle au moins 50 % et au plus 92 % du n-butane contenu dans celui-ci est transformé.

2. Procédé selon la revendication 1, **caractérisé par** :
- la transformation d'au moins une partie des hydrocarbures ramifiés contenus dans le courant partiel d'iso-C4 (i-C4) en hydrocarbures non ramifiés par isomérisation de squelette.

3. Procédé selon la revendication 1 ou 2, **caractérisé par** :
- la transformation d'au moins une partie du 1-butène contenu dans le courant d'hydrocarbures en C4 (C4) en 2-butène par hydroisomérisation avant l'obtention du courant partiel de n-C4 et d'iso-C4 (n-C4, i-C4) selon b) .

4. Procédé selon l'une quelconque des revendications précédentes, **caractérisé par** :
- le clivage à la vapeur d'au moins une partie du courant partiel d'iso-C4 (iso-C4) ou d'un courant dérivé de celui-ci avec une précision de clivage à laquelle plus de 91 % de l'isobutane contenu est transformé.

5. Procédé selon l'une quelconque des revendications précédentes, selon lequel, lors du clivage à la vapeur selon c), une précision de clivage à laquelle moins de 90 %, 88 %, 86 %, 84 %, 82 %, 80 %, 78 %, 76 %, 74 %, 72 %, 70 % ou 65 % et plus de 50 % ou 60 % du n-butane est transformé est utilisée.

6. Procédé selon l'une quelconque des revendications précédentes, selon lequel le courant partiel d'iso-C4 ou un courant dérivé de celui-ci (i-C4) est soumis au moins en partie à un procédé d'hydrogénation.

7. Procédé selon l'une quelconque des revendications précédentes, selon lequel le courant d'hydrocarbures en C4 (C4) préparé selon a) est formé au moins en partie à partir d'au moins un gaz de clivage (C) obtenu par clivage à la vapeur selon c).

8. Procédé selon l'une quelconque des revendications précédentes, selon lequel le courant d'hydrocarbures en C4 (C4) préparé selon a) est formé au moins en partie à partir d'un gaz de clivage (C), qui est formé par clivage à la vapeur d'une charge fraîche (A), notamment d'une ou de plusieurs fractions de pétrole, d'un ou de plusieurs composants de gaz naturel contenant deux à quatre atomes de carbone et/ou d'un ou de plusieurs condensats de gaz naturel.

9. Procédé selon l'une quelconque des revendications précédentes, selon lequel le courant d'hydrocarbures (C4) préparé selon a) est formé au moins en partie à partir d'une charge fraîche (A) non soumise auparavant à un procédé de clivage à la vapeur, notamment d'une ou de plusieurs fractions de pétrole, d'un ou de plusieurs composants de gaz naturel contenant deux à quatre atomes de carbone et/ou d'un ou de plusieurs condensats de gaz naturel et/ou d'au moins un produit d'un procédé de raffinerie.

10. Procédé selon l'une quelconque des revendications précédentes, selon lequel le clivage à la vapeur est réalisé en utilisant une quantité de vapeur de 0,4 kg/kg, notamment de 0,2 à 0,7 kg/kg.

11. Procédé selon l'une quelconque des revendications précédentes, selon lequel le clivage à la vapeur est réalisé dans au moins un four de clivage (11, 12, 13), dans lequel au moins une charge de four supplémentaire (A) sous la forme d'au moins un courant de recyclage et/ou d'au moins une charge fraîche est introduite.

12. Procédé selon l'une quelconque des revendications précédentes, selon lequel du 1,3-butadiène (BD) est séparé avant l'obtention du courant partiel de n-C4 et d'iso-C4 (i-C4, n-C4) selon b) à partir du courant d'hydrocarbures en C4 (C4).

13. Procédé selon la revendication 12, selon lequel l'isobutène contenu dans le courant d'hydrocarbures (C4) est au moins en partie transformé en un éther de tert-butyle après la séparation du 1,3-butadiène (BD) et l'éther de tert-butyle est également séparé du courant d'hydrocarbures (C4).

14. Procédé selon l'une quelconque des revendications précédentes, selon lequel au moins un courant supplémentaire, notamment un courant contenant des butynes et/ou des hydrocarbures à cinq atomes de carbone, est introduit dans le courant d'hydrocarbures en C4 (C4).
